# EUROPEAN PATENT APPLICATION

(11) **EP 1 647 256 A1**
(43) Date of publication of application: **19.04.2006**
(21) Application number: 03817419.9
(22) Date of filing: 09.07.2003
(51) Int. Cl.: A61K 6/00

(54) **DENDRITIC CELL INFILTRATIVITY ACTIVATING COMPOSITION AND IMMUNE ACTIVATOR**

(71) Applicant: Oncorex, Inc., Sapporo-shi, Hokkaido 060-0063 (JP)
(72) Inventor: KOBAYASHI, M., c/o Inst. for Genetic Med. Hokkido, Sapporo-shi, Hokkaido 060-08 (JP)
(74) Representative: Bassil, Nicholas Charles
(86) International application number: PCT/JP2003/008720
(87) International publication number: WO 2005/004926

(57) **Abstract**

The present invention's compositions for activating the infiltration activity of dendritic cells comprise retinoid. Retinoid increases the production of MMP-9, which is required for dendritic cells to exert their infiltration activity, and thereby activates the dendritic cells' infiltration activity. Therefore, the compositions exhibit immunopotentiating effects and can be used in the prevention and treatment of infectious diseases and cancers in animals.

SEQUENCE LISTING

<110> HOKKAIDO TECHNOLOGY LICENSING OFFICE Co. Ltd.

<120> dendritic cell infiltrativity activating composition and immune activator

<130> pctf158

<140>

<141>

<160> 8

<170> PatentIn Ver. 2.1

<210> 1

<211> 20

<212> DNA

<213> Artificial Sequence

<220>

<223> Description of Artificial Sequence:PCR primer

<400> 1

cctttgaggg cgacctcaag                                                    20

<210> 2

<211> 20

<212> DNA

<213> Artificial Sequence

<220>

<223> Description of Artificial Sequence:PCR primer

<400> 2

ctggatgacg atgtctgcgt 20

<210> 3

<211> 21

<212> DNA

<213> Artificial Sequence

<220>

<223> Description of Artificial Sequence:PCR primer

<400> 3

acagacggcc ttctgcaatt c 21

<210> 4

<211> 22

<212> DNA

<213> Artificial Sequence

<220>

<223> Description of Artificial Sequence:PCR primer

<400> 4

ggtgtagacg aaccggatgt ca                                                22

<210> 5

<211> 21

<212> DNA

<213> Artificial Sequence

<220>

<223> Description of Artificial Sequence:PCR primer

<400> 5

gttcaaaggg cctgagaagg a                                                 21

<210> 6

<211> 19

<212> DNA

<213> Artificial Sequence

<220>

<223> Description of Artificial Sequence:PCT primer

<400> 6

ccagggcacg atgaagtca                                                    19

<210> 7

<211> 20

<212> DNA

<213> Artificial Sequence

<220>

<223> Description of Artificial Sequence:PCR primer

<400> 7

gccttctgca actccgacat                                                    20

<210> 8

<211> 20

<212> DNA

<213> Artificial Sequence

<220>

<223> Description of Artificial Sequence:PCR primer

<400> 8

tctcggaagc ttccgtatgg                                                    20

## Description

### Technical Field

The present invention relates to compositions for activating the infiltration activity of dendritic cells, immunopotentiating agents, and such.

### Background Art

In conventional tumor immune therapy, various immune therapies have been tried, such as immunopotentiation therapy involving interleukin 2 (IL-2) administration or such, adoptive immunotherapy involving the infusion of lymphokine-activated killer (LAK) cells derived from a patient's peripheral lymphocytes or tumor infiltrating lymphocytes (TILs), and missile therapy using anti-tumor antigen monoclonal antibodies.

Furthermore, gene therapy for cancer has been actively performed. In particular, attention has been given to cell vaccine therapy that treats cancer by transplanting cancer cells introduced with cytokine genes, such as granulocyte-macrophage colony stimulating factor (GM-CSF), and inducing anti-cancer immunity. Dendritic cells are known as representative antigen-presenting cells. They are a population of cells that have dendritic morphology and are derived from hematopoietic stem cells. They are widely distributed in the body. The activation of helper T cells depends on antigen presentation by dendritic cells. Dendritic cells are thought to capture antigens in peripheral blood and migrate to lymph nodes, and eventually mature in lymph nodes.

In general, immature dendritic cells are very capable of ingesting foreign materials as antigens and digesting the ingested antigens, but they have low antigen-presenting ability. In contrast, mature dendritic cells are known to have low antigen-ingesting ability but high antigen-presenting ability. It is thought that, in the body, immature dendritic cells ingest foreign materials and digest these materials intracellularly; and mature dendritic cells which have differentiated as a result of the stimulation present a portion of the digested foreign materials extracellularly to transmit particular antigen information to lymphocytes.

Dendritic cells are known to be derived from hematopoietic stem cells of bone marrow in the body. Precursors of dendritic cells and immature dendritic cells exist in blood and lymph nodes, while mature dendritic cells exist in the spleen and lymph nodes. Mononuclear cells in peripheral tissues are known to differentiate into immature dendritic cells when stimulated with the granulocyte-macrophage colony stimulating factor (GM-CSF) and/or interleukin-4 (IL-4). In addition, immature dendritic cells are known to differentiate into mature dendritic cells when stimulated with the tumor necrosis factor (TNF).

Dendritic cells have the function of phagocytosing foreign materials that have invaded the body and presenting antigens to T cells. The T cells to which the antigens have been presented then phagocytose the antigens. To exert this function, dendritic cells travel from peripheral tissues to regional lymph nodes to show that foreign materials have invaded the body.

Generally, the oxygen level in cancer cells of cancer patients is reduced to about 20 % when compared with that in normal cells. The production of matrix metalloprotease-9 (MMP-9) that digests extracellular matrix proteins has been reduced in dendritic cells that differentiated under such hypoxic conditions. It is known that matrix metalloprotease-9 is required when dendritic cells migrate through peripheral tissues or pass through blood and lymphatic vessels in the body.

Thus, dendritic cells that have differentiated under hypoxic conditions cannot travel from peripheral tissues to lymph nodes and therefore cannot exert the antigen-presenting activity described above. Thus, dendritic cells cannot exert the antigen-presenting ability in tumor tissues, and therefore T cells cannot kill cancer cells.

Thus, one objective of the present invention is to provide compositions that allow dendritic cells produced under hypoxic conditions, such as those among cancer cells, to travel to lymph nodes, that is, to provide compositions that allow activation of the infiltration activity of dendritic cells. Another objective of the present invention is to provide immunopotentiating agents.

### Disclosure of the Invention

The present inventor conducted dedicated studies/research to achieve the objectives described above. As a result, the inventor discovered that retinoid activates the infiltration activity of dendritic cells and thus completed the present invention.

The present invention, which has been achieved on the basis of the finding described above, provides retinoid-comprising compositions for activating the infiltration activity of dendritic cells.

The present invention also provides methods for activating the infiltration activity of dendritic cells, which comprise retinoid administration to mammals.

The present invention also provides methods for activating the infiltration activity of dendritic cells, which comprise administration of the composition described above, which activate the infiltration activity of dendritic cells, to mammals.

The present invention also provides retinoid-comprising immunopotentiating agents.

The present invention also provides immunopotentiating agents comprising retinoid and dendritic cells.

The present invention also provides methods for potentiating immunity in mammals, which comprise retinoid administration into mammalian cells.

The present invention also provides methods for potentiating immunity in mammals, which comprise administration of the above-described immunopotentiating agent to mammals.

The present invention also provides preventive/therapeutic methods for infectious diseases and cancers in animals, which comprise administration or use of the above-described compositions for activating the infiltration activity of dendritic cells or the above-described immunopotentiating agents.

### Brief Description of the Drawings

Fig. 1 shows the result of surface antigen analysis by flow cytometry.
Fig. 2 shows the result of a real-time PCR analysis.
Fig. 3 shows the result of a Western blot analysis.
Fig. 4 shows the result of gelatin zymography.
Fig. 5 shows the result of gelatin zymography.
Fig. 6 is a graph showing the result of a real-time PCR analysis.
Fig. 7 is a graph showing the result of measuring the infiltration activities of immature and mature dendritic cells.
Fig. 8 is a graph showing a result obtained from analysis of changes in the infiltration activity of dendritic cells induced by TIMP-1 protein.
Fig. 9 is a graph showing the result of measuring MMP-9 mRNA levels in cells after the induction of differentiation.
Fig. 10 shows the result of measuring the infiltration activity of dendritic cells that have differentiated under hypoxic conditions.

### Best Mode for Carrying out the Invention

First, the compositions of the present invention for activating the infiltration activity of dendritic cells are described below.

The compositions of the present invention for activating the infiltration activity of dendritic cells comprise retinoid. The retinoids to be used in the compositions of the present invention for activating the infiltration activity of dendritic cells include, for example, retinoic acids, retinals, retinols, and retinyl fatty acid esters, as well as dehydroretinols, dehydroretinols, and dehydroretinyl fatty acid esters.

The above-described retinoic acids have the following retinoic acid isomers: all-trans retinoic acid, 13-*cis*-retinoic acid, 11-*cis*-retinoic acid, 9-*cis*-retinoic acid, 3,4-dehydroretinoic acid, and such.

Retinols have the following isomers: all-trans retinol, 13-*cis*-retinol, 11-*cis*-retinol, 9-*cis-*retinol, 3,4-dehydroretinol, and such. All-trans retinol, 13-*cis*-retinol, all-trans retinoic acid, and 13-*cis*-retinoic acid are preferred because they are widely available on the market.

The retinoid content in the compositions of the present invention for activating the infiltration activity of dendritic cells is defined as the dose required for the activation of the infiltration activity of dendritic cells when administered to patients who require the treatment. The dose to be administered to a patient is generally determined on the basis of the patient's body surface area, weight, disease conditions, or such. The dose of the compositions of the present invention for activating the infiltration activity of dendritic cells is about 20 to about 50 mg/m² in terms of the retinoid amount. It is preferable to adjust the dose depending on the type of administration method, the amount of excipient, or whether other agents are used in combination.

The compositions of the present invention for activating the infiltration activity of dendritic cells may be administered parenterally, for example, subcutaneously, intraperitoneally, intramuscularly, and intravenously. Examples of a parenteral preparation form include an aqueous solution comprising an activator and ~5% glucose or another known pharmaceutically acceptable excipient in an isotonic solution. Solubilizing agents such as cyclodextrin or other solubilizing agent known to those skilled in the art may also be added as an excipient.

Next, the immunopotentiating agents of the present invention are described below.

The immunopotentiating agents of the present invention comprise retinoid. Retinoids used in the above-described compositions that activate the infiltration activity of dendritic cells may also be used as retinoids in the immunopotentiating agents of the present invention.

The retinoid content of the immunopotentiating agents, dosage, administration form, and such are the same as those described above for the compositions for activating the infiltration activity of dendritic cells.

The compositions for activating the infiltration activity of dendritic cells and the immunopotentiating agents in the present invention enhance the production of MMP-9, a protein required for the infiltration of dendritic cells, and thus have an effect in activating the infiltration activity of dendritic cells. The compositions and agents exert immunopotentiation activity by activating the infiltration activity of dendritic cells and thus migration of the dendritic cells to lymph nodes. The compositions for activating the infiltration activity of dendritic cells and immunopotentiating agents in the present invention are used for mammals (e.g., mice, cats, dogs, cows, horses, sheep, goats, rabbits, and humans) because they have this effect. The compositions and agents can be used in the treatment and/or prevention of various leukemias; various cancers, such as malignant lymphoma, pancreatic cancer, hepatic cancer, lung cancer, stomach cancer, colon cancer, osteogenic sarcoma, malignant melanoma, malignant chorioepithelioma, myosarcoma, ovarian cancer, uterine cancer, prostatic carcinoma, esophageal cancer, head and neck tumors, and brain tumors; infectious diseases, such as those caused by various viruses and bacteria; and such.

The immunopotentiating agents of the present invention may further comprise dendritic cells.

Dendritic cells can be obtained by culturing mononuclear cells, which are a precursor of dendritic cells and can be obtained by using peripheral blood, bone marrow fluid, cord blood, or such as a raw material. Dendritic cells that are used in the immunopotentiating agents of the present invention may be mature or immature. For example, mononuclear cells can be induced to differentiate into mature dendritic cells through stimulation with GM-CSF, IL-4 and TNF-α, and culture.

Dendritic cells that are used for the immunopotentiating agents of the present invention can be obtained by culturing the above-described mononuclear cells in the presence of a differentiation-inducing agent. Mononuclear cells in peripheral blood, for example, can be used as a raw material for the dendritic cells. Mononuclear cells, a precursor of dendritic cells, can be prepared by, for example, collecting blood and isolating and purifying mononuclear cells from the collected blood. There is no limitation on the type of method for separating mononuclear cells from erythrocytes; and conventionally known methods may be used for this purpose.

For example, methods utilizing Ficoll-Paque density gradient or elution are generally used for separating peripheral blood mononuclear cells. Alternatively, the separation can be achieved by suspending blood cells in a solution that selectively lyses adult erythrocytes, for example, ammonium chloride-potassium and ammonium oxalate.

A suitable commercially available medium, such as RPMI-1640, Dulbecco's modified Eagle medium (DMEM), or Iscove's medium (IMDM), can be used in the culture to induce the differentiation of dendritic cells. Serum may be added to the culture medium; for example, about 5 to 20% bovine serum, fetal calf serum, or human serum may be used. The culture can be carried out without serum; alternatively bovine serum albumin (BSA), human serum albumin (HSA), or the like may be added if required. The medium may contain an appropriate antibiotic, an antibody, pyruvic acid (about 0.1 to 5 mM), glutamine (about 0.5 to 5 mM), and/or 2-mercaptoethanol (about 10 to 100 µM), as needed. A differentiation-inducing agent is added to the culture medium, and the incubation is carried out at about 37°C in about 5% carbon dioxide atmosphere for about 5 to 21 days. The incubation period is preferably in the range of about 7 to 14 days. The culture temperature (34 to 38 °C) and gas mixing ratio (2 to 10% carbon dioxide gas; nitrogen or oxygen gas may be mixed thereto) can be appropriately selected.

Use of an appropriate inducer is required for inducing the differentiation of dendritic cells from a group of cells comprising precursors of dendritic cells. Cytokines can be used as an inducer. An appropriate cytokine may be used. For example, GM-CSF, IL-4, stem cell factor (SCF), interleukin-13 (IL-13), TNF-α, or Flt3 ligand may be used. The concentrations of the above-described cytokines in a culture medium are preferably in the range of about 1 to 1000 ng/ml.

The cytokines used in the culture may be a factor derived from heterologous animals, such as mice, but a factor derived from humans is preferred.

Dendritic cells used in the immunopotentiating agents of the present invention are administered into human bodies, and therefore it is safer to use cells whose cell proliferation ability has been abolished. It is known that induction of the differentiation of mononuclear cells reduces their proliferation ability. For the safer use of the cells, it is preferable to pretreat them by heating or irradiation, or with mitomycin C.

For example, when carrying out X-ray irradiation, it is preferred that a container containing dendritic cells be placed under an X-ray irradiation tube and then X-ray irradiated at a total radiation dose of about 1000 to 3300 rad. Mitomycin C treatment is carried out, for example, by the following procedure: preparing a suspension of dendritic cells by suspending them at a cell density of 1 to 3x 10⁷ cells/ml, adding mitomycin C to the cell suspension at a ratio of 25 to 50 µg/ml, and incubating the suspension at 37°C for about 30 to 60 minutes. Heat treatment is carried out, for example, by the following procedure: preparing a suspension of dendritic cells by suspending them at a cell density of about 1x 10⁷ cells/ml, and heating the cell suspension at 50 to 65°C for about 20 minutes.

The dendritic cell-comprising immunopotentiating agents of the present invention described above exert their immunopotentiating activity through activation of the infiltration activity of dendritic cells by retinol. Thus, the above-described immunopotentiating agents are used for various leukemias; various cancers, such as malignant lymphoma, pancreatic cancer, hepatic cancer, lung cancer, stomach cancer, colon cancer, osteogenic sarcoma, malignant melanoma, malignant chorioepithelioma, myosarcoma, ovarian cancer, uterine cancer, prostatic carcinoma, esophageal cancer, head and neck tumors, and brain tumors; infectious diseases, such as those caused by various viruses and bacteria; and such in mammals (for example, mice, cats, dogs, cows, horses, sheep, goats, rabbits, humans, and others). The dosage of the dendritic cell-comprising immunopotentiating agents cannot be flatly determined because it varies depending on the patient's age, weight, and sex, as well as symptoms, type, and stage of cancer, and such. As an example, dendritic cells can be administered at a dose of 1x 10⁷ cells per patient once a week for ten weeks.

Next, the methods of the present invention for activating the infiltration activity of dendritic cells are described below.

The methods of the present invention for activating the infiltration activity of dendritic cells comprise retinoid administration to mammals. The retinoids to be used in the methods of the present invention for activating the infiltration activity of dendritic cells include, for example, retinoic acids, retinals, retinols, retinyl fatty acid esters, dehydroretinols, dehydroretinols, and dehydroretinyl fatty acid esters.

The retinoic acids described above have, for example, the following retinoic acid isomers: all-trans retinoic acid, 13-*cis*-retinoic acid, 11-*cis*-retinoic acid, 9-*cis*-retinoic acid, and 3,4-dehydroretinoic acid.

Retinols have, for example, the following retinol isomers: all-trans retinol, 13-*cis*-retinol, 11-*cis*-retinol, 9-*cis*-retinol, 3,4-dehydroretinol. All-trans retinol, 13-*cis*-retinol, all-trans retinoic acid, and 13-*cis*-retinoic acid are preferred because of their wide commercial availability.

The above-described compositions of the present invention for activating the infiltration activity of dendritic cells can be used as retinoids in the methods of the present invention for activating the infiltration activity of dendritic cells.

The present invention's methods for activating the infiltration activity of dendritic cells are applicable to mammals including mice, cats, dogs, cows, horses, sheep, goats, rabbits, humans, and others. The retinoid dosage used in the methods of the present invention for activating the infiltration activity of dendritic cells is defined as the dosage required for the activation of the infiltration activity of dendritic cells when administered to patients in need of the treatment. The dosage to be administered to a patient is generally determined on the basis of the patient's body surface area, weight, state of disease, or such. The dosage of the compositions of the present invention for activating the infiltration activity of dendritic cells is in the range of about 20 to about 50 mg/m² in terms of the amount of retinoid. It is preferable to adjust the dosage depending on the type of administration method, amount of excipient, or whether other agents are used in combination. The compositions are preferably administered parenterally, for example, subcutaneously, intraperitoneally, intramuscularly, or intravenously.

By using the present invention's methods for activating the infiltration activity of dendritic cells, the immunopotentiating activity is exerted as the production of MMP-9, a protein required for the infiltration of dendritic cells, is enhanced, and the infiltration activity of dendritic cells and their migration to the lymph nodes are activated. Thus, the methods of the present invention for activating the infiltration activity of dendritic cells are applicable to the mammals described above for the treatment and/or prevention of various leukemias; various cancers, such as malignant lymphoma, pancreatic cancer, hepatic cancer, lung cancer, stomach cancer, colon cancer, osteogenic sarcoma, malignant melanoma, malignant chorioepithelioma, myosarcoma, ovarian cancer, uterine cancer, prostatic carcinoma, esophageal cancer, head and neck tumors, and brain tumors; infectious diseases, such as those caused by various viruses and bacteria; and such. Specifically, the present invention provides preventive and/or therapeutic methods for infectious diseases or cancers in animals, which comprise the administration or use of the compositions of the present invention for activating the infiltration activity of dendritic cells.

Next, methods for potentiating immunity in the present invention are described below.

The present invention's methods for potentiating immunity comprise retinoid administration to mammals. The retinoids to be used in the present invention's methods for potentiating immunity include, for example, retinoic acids, retinals, retinols, retinyl fatty acid esters, dehydroretinols, dehydroretinols and dehydroretinyl fatty acid esters.

The above-described retinoic acids have, for example, the following isomers: all-trans retinoic acid, 13-*cis*-retinoic acid, 11-*cis*-retinoic acid, 9-*cis*-retinoic acid, and 3,4-dehydroretinoic acid.

Retinols have, for example, the following retinol isomers: all-trans retinol, 13-*cis*-retinol, 11-cis-retinol, 9-*cis*-retinol, 3,4-dehydroretinol. All-trans retinol, 13-*cis*-retinol, all-trans retinoic acid, and 13-*cis*-retinoic acid are preferred because of their wide commercial availability.

The immunopotentiating agents of the present invention described above can be used as a retinoid in the present invention's methods for potentiating immunity.

Mammals to which the present invention's methods for potentiating immunity are applicable include the above-described mammals to which the present invention's methods for activating the infiltration activity of dendritic cells are applicable. The retinoid dosage to be used, its administration method, and such are the same as those described above for the methods for activating the infiltration activity of dendritic cells.

By using the methods of the present invention for potentiating immunity, the immunopotentiating activity is exerted as the production of MMP-9, a protein required for the infiltration of dendritic cells, is enhanced, and the infiltration activity of dendritic cells and their migration to the lymph nodes are activated. Thus, the methods of the present invention for potentiating immunity are applicable to the mammals described above for the treatment and/or prevention of various leukemias; various cancers, such as malignant lymphoma, pancreatic cancer, hepatic cancer, lung cancer, stomach cancer, colon cancer, osteogenic sarcoma, malignant melanoma, malignant chorioepithelioma, myosarcoma, ovarian cancer, uterine cancer, prostatic carcinoma, esophageal cancer, head and neck tumors, and brain tumors; infectious diseases, such as those caused by various viruses and bacteria; and such. Specifically, the present invention provides preventive and/or therapeutic methods for infectious diseases or cancers in animals, which comprise the administration or use of the immunopotentiating agents of the present invention.

### Examples

The present invention is illustrated in detail below with reference to Examples, but is not to be construed as being limited thereto.

### [Example 1]

Peripheral blood mononuclear cells (PBMCs) from healthy subjects were separated by Ficoll-Paque density gradient centrifugation. Peripheral blood mononuclear cells were dispersed at a cell density of 2x 10⁶ cells/ml in complete RPMI-1640e medium, and the resulting suspension is incubated at 37°C for one hour to give adhesive cells. Nonadhesive cells were removed by washing five times with RPMI-1640 medium. The adhesive cells were cultured in RPMI medium containing 1000 U/ml GM-CSF and 1000 U/ml IL-4, 10% FBS, 25 mM HEPES, 2 mM L-glutamic acid, 50 µM 2-ME, 1% nonessential amino acids, and 2 mM sodium pyruvate under hypoxic conditions (oxygen concentration was 1%; herein, hypoxic conditions refer to conditions where the oxygen concentration is 1%) or normoxic conditions (oxygen concentration was 20%; herein, normoxic conditions refer to conditions where the oxygen concentration is 20%) at 37°C under an 5% carbon dioxide atmosphere for six days with half of the medium being changed every day. On day six, the cells were collected and divided into two groups. The first group was cultured in a medium containing IL-4 and GM-CSF for one day, and then used as immature dendritic cells. The second group was treated with LPS (1 µg/ml) for one day and then used as mature dendritic cells.

### [Example 2]

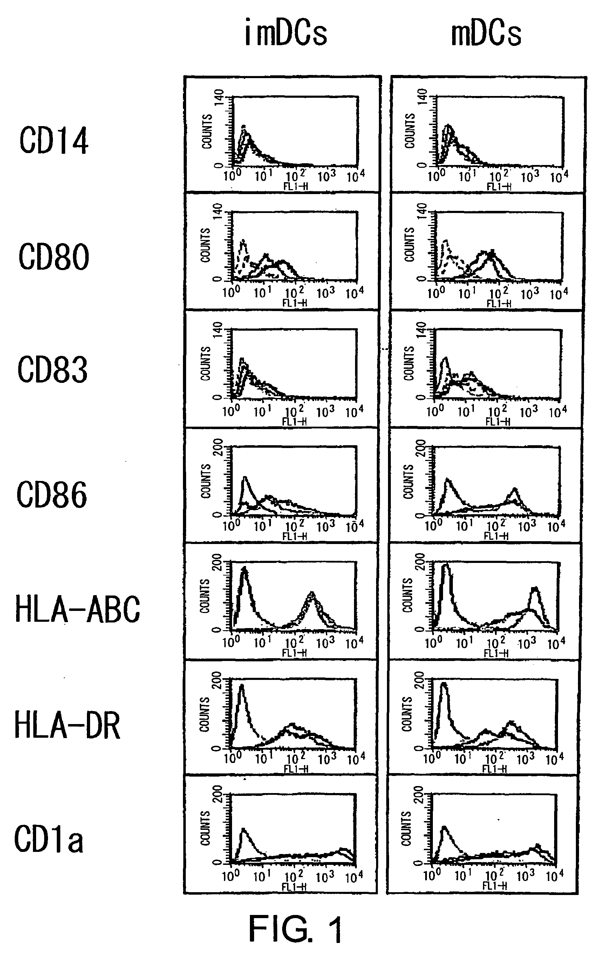

Surface antigens on dendritic cells obtained in Example 1 were detected by flow cytometry. Antigen detection was performed for immature and mature dendritic cells. The cells (5x 10⁵ cells) to be assayed were incubated with an FITC-conjugated mouse monoclonal antibody against CD83, CD80, CD14, HLA-ABC, or HLA-DR, or a mouse monoclonal antibody against human CD1a or CD86 at 4°C for 30 minutes. The monoclonal antibodies used were purchased from Immunotech. Then, the cells were washed twice with phosphate-buffered physiological saline (PBS, pH 7.0) to remove excess monoclonal antibodies. The cells were then incubated with a fluorescein-conjugated goat anti-mouse IgG + IgM(H+L) antibody (Jackson ImmunoResearch Laboratories) for 45 minutes, and analyzed by flow cytometry. The result is shown in Fig. 1. Fig. 1 shows a result of the analysis of surface antigens by flow cytometry, where imDCs and mDCs represent immature and mature dendritic cells, respectively.

As shown in Fig. 1, CD14, a monocyte marker, was expressed in neither immature nor mature dendritic cells. A dendritic cell marker CD83 was expressed in immature and mature dendritic cells. Costimulatory molecules CD80 and CD86 were expressed in immature and mature dendritic cells. HLA-DR, HLA-ABC, and CD1a were expressed in immature and mature dendritic cells. From these results, the cells which had differentiated in the presence of IL-4 and GM-CSF under normoxic or hypoxic conditions were judged to be immature dendritic cells (imDCs), whereas the LPS-treated cells that had differentiated under normoxic or hypoxic conditions were judged to be mature dendritic cells (mDCs).

### [Example 3]

The immature and mature dendritic cells obtained in Example 1 were induced to differentiate under hypoxic or normoxic conditions, and the cellular expressions of MMP-9 mRNA were examined by real-time PCR. Differentiations under hypoxic or normoxic conditions were carried out under 1% and 20% oxygen concentrations, respectively, in a culture chamber for seven days. Real-time PCR was carried out by the following procedure. The cells used were immature and mature dendritic cells derived from three donors. Total RNA was isolated from the cultured cells (1x 10⁶ cells) using TRIzol reagent (Life Technologies). Each RNA sample (2 µg) was converted to cDNA in 50 µl of reaction mixture containing 75 mM KCl, 50 mM Tris-HCl (pH 8.3), 3 mM MgCl₂, 10 mM dithiothreitol, 0.5 mM each of dNTPs, 2 µM random primer, and 1000 U of TaqMan Reverse Transcription reagent. Each cDNA (10 ng) was amplified in triplicate using an SYBR-Green PCR assay kit, and sequenced using an ABI PRISM 7900HT sequence Detection System. PCR was carried out by incubation at 50°C for 2 minutes and then at 95 °C for 15 minutes, followed by 15 cycles of denaturation at 95 °C, 30 cycles of annealing at 60°C, and extension at 72°C for 1 minute. The sequences shown in SEQ ID NOs: 1 and 2 were used as the primers.

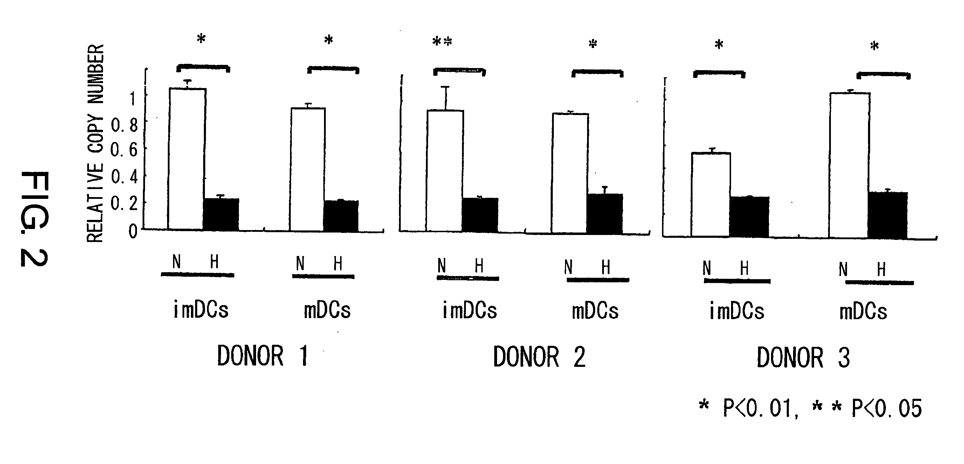

The result is shown in Fig. 2. Fig. 2 shows a result obtained by real-time PCR, where imDCs and mDCs represent immature and mature dendritic cells; and N and H represent normoxic and hypoxic conditions, respectively. As shown in Fig. 2, immature and mature dendritic cells that differentiated under normoxic condition expressed MMP-9 mRNA at levels two- to five-fold higher than those that differentiated under hypoxic condition.

### [Example 4]

The immature and mature dendritic cells obtained in Example 1 were diluted to 5x 10⁵ cells/ml with serum-free RPMI1640 medium, and then cultured at 37°C under hypoxic or normoxic conditions for 24 hours. Each culture medium was centrifuged and the resulting supernatant was collected and stored at -70°C. To quantify the MMP-9 protein produced, supernatants of the dendritic cell culture having the same protein concentrations were loaded onto 7.5 W/V polyacrylamide gel to carry out SDS-PAGE. The culture medium of the human fibrosarcoma cell line TH 1080 was used as positive control (which produces β-actin). After SDS-PAGE, proteins were transferred onto a nitrocellulose filter (Bio-Rad, Hercules, CA) by semidry blotting. After blocking and washing were carried out by a standard procedure, the filter was incubated with 1 µg/ml primary antibody against human MMP-9 at room temperature for one hour. The primary antibody used was mouse polyclonal IgG produced against human MMP-9, while the secondary antibody was 1:2000 diluted horseradish peroxidase-labeled rabbit anti-mouse IgG (H&L). To detect MMP-9 protein in cells, each cell lysate containing 15 µg of proteins was loaded and analyzed by Western blotting. The filter was incubated with peroxidase-labeled goat anti-mouse IgG, and developed using an ELC detection kit (Amersham, Tokyo, Japan).

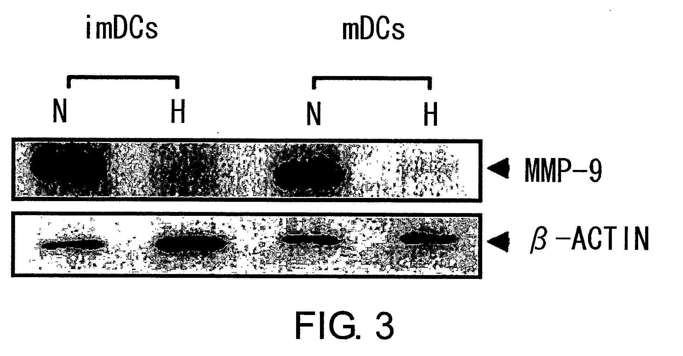

The result is shown in Fig. 3. Fig. 3 shows a result obtained by Western blotting. As shown in Fig. 3, the MMP-9 protein was produced at higher levels in immature and mature dendritic cells that differentiated under normoxic conditions than in dendritic cells that differentiated under hypoxic conditions.

### [Example 5]

The immature and mature dendritic cells cultured in Example 4 were analyzed by gelatin zymography. The method is described below.

The assay was carried out by electrophoresis using polyacrylamide gel containing sodium dodecyl sulfate (SDS) and gelatin. Briefly, culture supernatants separated from the cell cultures having the same protein composition were combined with three volumes of buffer, and electrophoresed on a 10% W/V polyacrylamide gel containing 1 mg/ml gelatin. After electrophoresis, the gel was incubated in 100 ml of recycled buffer (solution of 2.5% Triton X-100 in deionized water) with stirring. Then, the gel was incubated in 100 ml of development buffer (0.15 M NaCl, 50 mM Tris-HCl, 10 mM CaCl₂, and 0.05% NaN₃) at 37°C overnight. The gel was stained with 0.5% Coomassie Blue R-250 in 40% methanol/10% acetic acid solution at room temperature for one hour. Then, the gel was destained for four hours with occasional changes of the destaining solution (40% methanol/10% acetic acid in deionized water). Clear bands were distinctly seen in the dark-blue background, representing proteolytic activity. The culture medium of the human fibrosarcoma cell line TH 1080 was used as positive control.

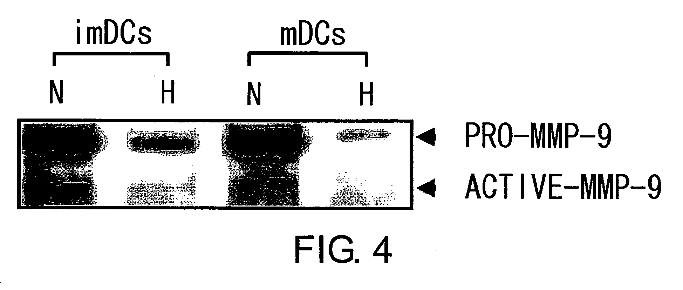

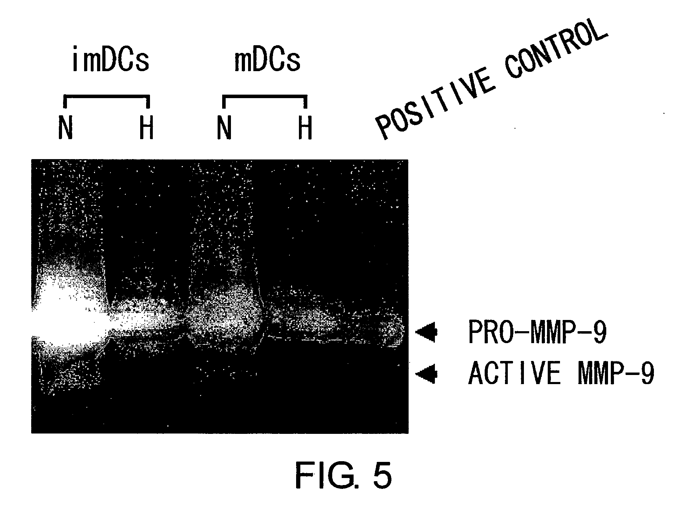

As shown in Fig. 4, immature and mature dendritic cells that differentiated under normoxic conditions secreted the MMP-9 protein at higher levels than those that differentiated under hypoxic conditions. The active form of MMP-9 was detected in only dendritic cells that differentiated under normoxic conditions. Then, immature and mature dendritic cells that differentiated under normoxic or hypoxic conditions were tested for the proteolytic activity of the MMP-9 protein secreted from the cells. As shown in Fig.5, the gelatin zymograph shows that the dendritic cells that differentiated under normoxic conditions secreted pro-MMP-9 at higher levels than those that differentiated under hypoxic conditions. The active form of the MMP-9 protein was only secreted in dendritic cells that differentiated under normoxic condition. These results show that the proteolytic activity is lower in dendritic cells that differentiated under hypoxic conditions than in those that differentiated under normoxic conditions.

### [Example 6]

The immature and mature dendritic cells obtained in Example 1 were induced to differentiate under hypoxic or normoxic conditions, and the expression of the MMP-9-specific inhibitors TIMP-1, TIMP-2, and TIMP-3 in each type of cells was examined by real-time PCR. The method used was the same as that described in Example 3. As primers for TIMP-1, sequences shown in SEQ ID NOs: 3 and 4 were used; as primers for TIMP-2, those shown in SEQ ID NOs: 5 and 6; and as primers for TIMP-3, those shown in SEQ ID NOs: 7 and 8.

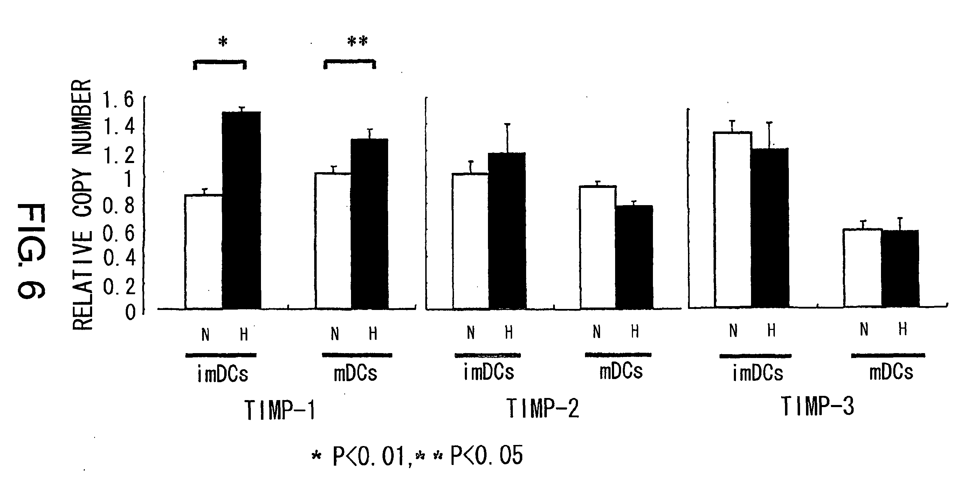

The result is shown in Fig. 6. Fig. 6 shows a result of real-time PCR. As shown in Fig. 6, TIMP-1 was expressed at higher levels in dendritic cells that differentiated under hypoxic conditions than in those that differentiated under normoxic conditions. TIMP-2 and TIMP-3 were expressed at the same level in dendritic cells cultured under normoxic and hypoxic conditions. TIMP-1 is an MMP-9-specific inhibitor, and this result shows that the proteolytic activity of dendritic cells that differentiated under hypoxic conditions is lower than that of dendritic cells that differentiated under normoxic conditions.

### [Example 7]

The infiltration activity of dendritic cells was measured by the Transwell Chamber method using the following procedure: a Matrigel was immobilized onto a membrane that separates a top chamber and a bottom chamber; after washing, dendritic cells were placed in the top chamber; 100 ng/ml recombinant human RANTES was placed in the bottom chamber when immature dendritic cells were used, or 100 ng/ml recombinant human MCP-3 was placed in the bottom chamber when mature dendritic cells were used; cells migrating into the bottom chamber were counted.

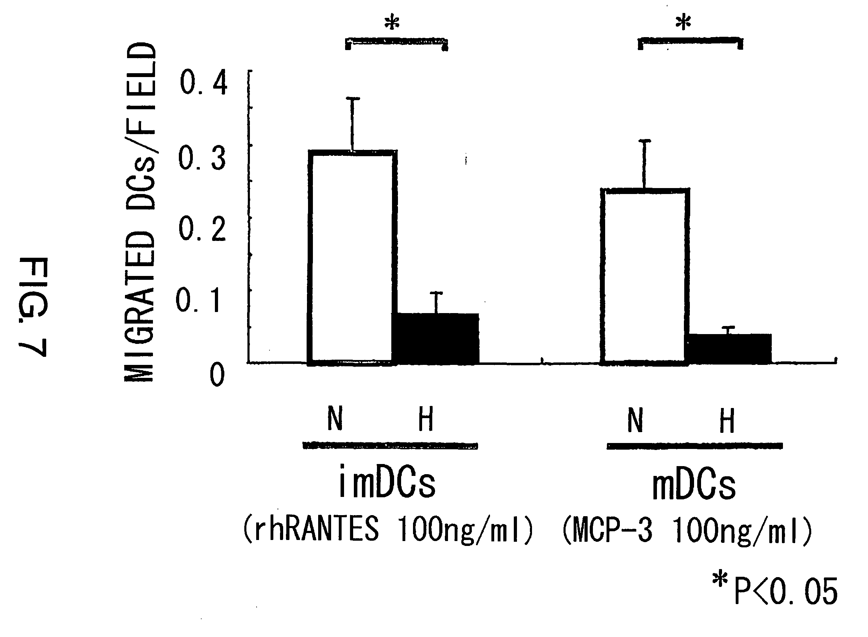

The result is shown in Fig. 7. Fig. 7 is a graph showing a result of the measurement of the infiltration activities of immature and mature dendritic cells. As shown in Fig. 7, both immature and mature dendritic cells exhibited higher infiltration activities when they differentiated under normoxic conditions than when they differentiated under hypoxic conditions.

### [Example 8]

To evaluate whether the infiltration activity of dendritic cells is reduced by TIMP-1 protein, 10 ng/ml TIMP-1 protein was added to the same system as described in Example 7, and the same assay was carried out.

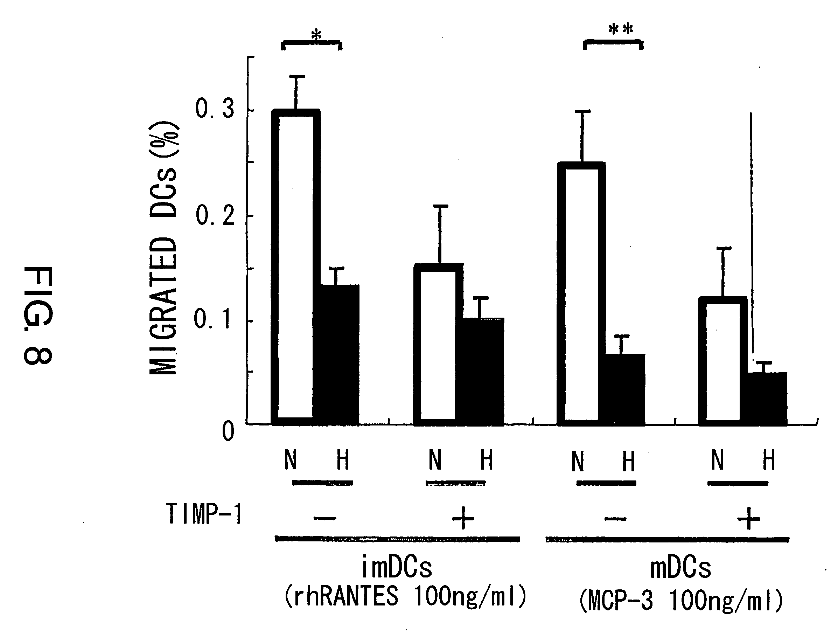

The result is shown in Fig. 8. Fig. 8 is a graph showing a result obtained by analyzing the changes in the infiltration activity of dendritic cells induced by the TIMP-1 protein. In Fig. 8, + indicates the result obtained when the TIMP-1 protein was added, and ― indicates the result obtained when the TIMP-1 protein was not added. As shown in Fig. 8, the infiltration activity of immature and mature dendritic cells that differentiated under normoxic conditions was reduced by the TIMP-1 protein. In contrast, the infiltration activity of immature and mature dendritic cells that differentiated under hypoxic conditions was not changed by the TIMP-1 protein.

### [Example 9]

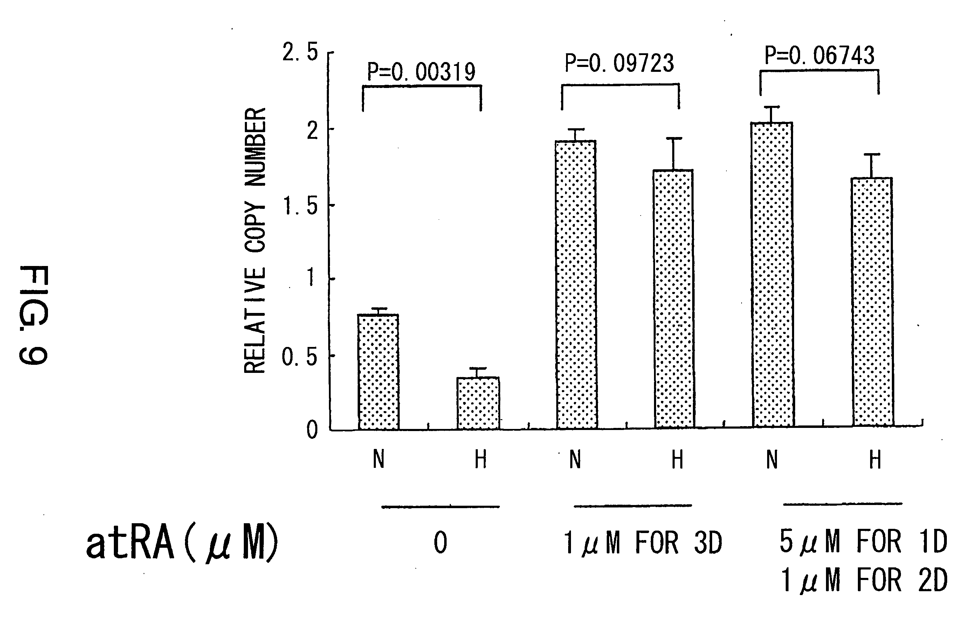

The mature dendritic cells obtained in Example 1 were cultured in the culture medium supplemented with 1 µM all-trans retinoic acid for three days or in the culture medium supplemented with 5 µM all-trans retinoic acid for one day. The cells were further cultured in the culture medium supplemented with 1 µM all-trans retinoic acid for two days, to induce the differentiation of the dendritic cells. After the induction of the differentiation, MMP-9 mRNA level in the cells was determined. The result is shown in Fig. 9. As shown in Fig. 9, MMP-9 mRNA level was increased with the addition of all-trans retinoic acid to the culture medium.

### [Example 10]

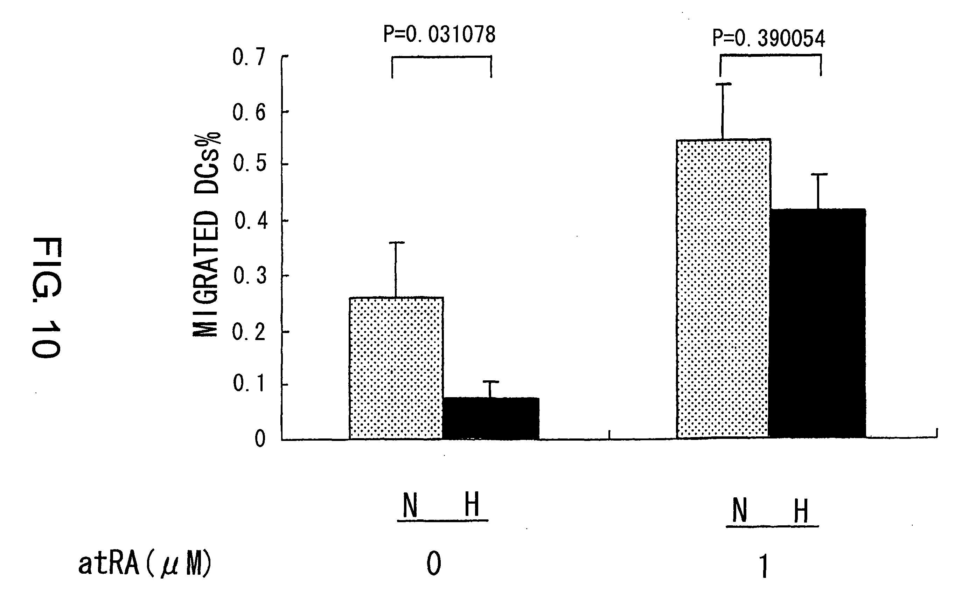

The mature dendritic cells obtained in Example 1 were cultured in the culture medium supplemented with 1 µM all-trans retinoic acid to induce their differentiation. Then, the infiltration activity of dendritic cells was determined by the same procedure described in Example 7. The result is shown in Fig. 10. Fig. 10 shows a result of the measurement of the infiltration activity of dendritic cells that differentiated under hypoxic conditions. As shown in Fig. 10, the infiltration activity of dendritic cells was increased with the addition of all-trans retinoic acid to the culture medium.

As described in detail above, all-trans retinoic acid, a retinoid, was found to enhance the production of MMP-9, which is required for dendritic cells to exert their infiltration activity, and thereby activates the infiltration activity of dendritic cells. This effect is exerted even under hypoxic conditions, and even in tumor tissues in which oxygen level is reduced. Thus, the compositions of the present invention for activating the infiltration activity of dendritic cells exert immunopotentiating activity, and can also be used to potentiate immunity in mammals. In addition, the present invention's compositions for activating the infiltration activity of dendritic cells and the immunopotentiating agents can also be used in the prevention and/or treatment of infectious diseases and cancers in animals.

## Claims

1. A retinoid-comprising composition for activating infiltration activity of dendritic cells.

2. The composition for activating infiltration activity of dendritic cells according to claim 1, wherein the retinoid is selected from the group consisting of all-trans retinol, 13-*cis*-retinol, all-trans retinoic acid, and 13-*cis*-retinoic acid.

3. A method for activating infiltration activity of dendritic cells, which comprises administering retinoid to a mammal.

4. The method for activating infiltration activity of dendritic cells according to claim 3, wherein the retinoid is selected from the group consisting of all-trans retinol, 13-*cis*-retinol, all-trans retinoic acid, and 13-*cis*-retinoic acid.

5. A method for activating infiltration activity of dendritic cells, which comprises administering to a mammal the composition for activating the infiltration activity of dendritic cells according to claim 1 or 2.

6. An immunopotentiating agent comprising retinoid.

7. An immunopotentiating agent comprising retinoid and a dendritic cell.

8. The immunopotentiating agent according to claim 6 or 7, wherein the retinoid is selected from the group consisting of all-trans retinol, 13-*cis*-retinol, all-trans retinoic acid, and 13-*cis*-retinoic acid.

9. A method for potentiating immunity in a mammal, which comprises introducing retinoid to a mammalian cell.

10. A method for potentiating immunity in a mammal, which comprises administering to the mammal the immunopotentiating agent according to any one of claims 6 to 8.

11. The method for potentiating immunity in a mammal according to claim 10, wherein the retinoid is selected from the group consisting of all-trans retinol, 13-*cis*-retinol, all-trans retinoic acid, and 13-*cis*-retinoic acid.

12. A method for preventing or treating infectious disease or cancer in an animal, which comprises administering or using the composition for activating infiltration activity of dendritic cells according to claim 1 or 2, or the immunopotentiating agent according to any one of claims 6 to 8.
